Europäisches Patentamt

European Patent Office

Office européen des brevets

(11) Veröffentlichungsnummer: **0 243 295 B1**

(12) **EUROPÄISCHE PATENTSCHRIFT**

(45) Veröffentlichungstag der Patentschrift: **13.05.92**

(21) Anmeldenummer: **87730029.3**

(22) Anmeldetag: **18.03.87**

(51) Int. Cl.⁵: **A61M 13/00**

(54) **Vorrichtung zum Insufflieren von Gas.**

(30) Priorität: **27.03.86 DE 3611018**

(43) Veröffentlichungstag der Anmeldung:
**28.10.87 Patentblatt 87/44**

(45) Bekanntmachung des Hinweises auf die
Patenterteilung:
**13.05.92 Patentblatt 92/20**

(84) Benannte Vertragsstaaten:
**CH ES FR GB IT LI NL SE**

(56) Entgegenhaltungen:
**DE-A- 3 413 631**
**US-A- 4 464 169**

(73) Patentinhaber: **Wiest, Peter P., Dipl.-Ing.**
**Hessenallee 8**
**W-1000 Berlin 19(DE)**

(72) Erfinder: **Wiest, Peter P., Dipl.-Ing.**
**Hessenallee 8**
**W-1000 Berlin 19(DE)**
Erfinder: **Fuchs, Hubert G., Dipl.-Ing.**
**Grunewaldstrasse 13**
**W-1000 Berlin 41(DE)**

(74) Vertreter: **Lüke, Dierck-Wilm, Dipl.-Ing.**
**Gelfertstrasse 56**
**W-1000 Berlin 33(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Vorrichtung zum Insufflieren von Gas gemäß dem Oberbegriff des Anspruches 1.

Bei einer aus der DE-OS 34 13 631 vorbekannten Vorrichtung dieser Art sind zwei festeingestellte Druckminderer vorgesehen, die den Gasdruck z.B. auf 50 mmHg festeingestellt vorgeben. Ein nachgeschalteter Durchflußregler regelt die Strömung in der Gasleitung z.B. auf 1 l/min. Das Gas strömt durch ein Magnetventil in einen Durchflußmesser, wobei der hier infolge Reibungswiderständen entstehende Druckabfall mit Hilfe des Durchflußreglers konstant gehalten wird. Der Druckabfall dient zugleich der Durchflußregelung im Durchflußregler. Das Gas durchströmt sodann eine optische Durchflußanzeigeeinrichtung, bevor es in das Insufflationsinstrument einströmen kann. Unmittelbar davor ist der Druckmesser an die Gasleitung angeschlossen. Die bekannte Vorrichtung umfaßt ferner neben der Haupt-Gasleitung eine Zweigleitung, die den Durchflußregler über ein Magnetventil mit dem optischen Durchflußanzeiger verbindet. Die beiden Magnetventile, das Druckmessgerät und der Durchflußmesser sind mit einer elektronischen Auswerte- und Steuerschaltung verbunden, welche mit einer Anzeigevorrichtung für den Gesamtdruck, den intraabdominalen statischen Druck, die Gasströmungsgeschwindigkeit und die insgesamt verbrauchte Gasmenge versehen ist.

Die bekannte Vorrichtung ist aufgrund der Vielzahl von Bauelementen aufwendig und teuer in der Herstellung. Ferner befinden sich die beiden Magnetventile , die beiden Durchflußmesser bzw.- regler und der optische Durchflußanzeiger in der Gasleitung, wodurch Störungen, insbesondere erhebliche Widerstände in der Gasströmung, verursacht werden. Schließlich ist das mit dieser Vorrichtung durchzuführende Verfahren der Druckmessung und der Flowregelung sehr kompliziert. Zunächst muß beim Einschalten der Vorrichtung der Strömungswiderstand des gesamten Leitungssystemes ermittelt werden, welcher Widerstandsdruck zur Bestimmung des intraabdominalen statischen Druckes vom momentan am Druckmeßgerät gemessenen Druckwert abgezogen werden muß, so daß die Differenz beider Druckwerte den intraabdominalen statischen Druck angibt. Ferner wird bei Erreichen eines vorgewählten Solldrucks der Gasfluß automatisch durch die elektronische Steuerschaltung gestoppt, wobei die durch den Durchflußmesser führende Haupt-Gasleitung geschlossen und die Zweigleitung synchron geöffnet wird. Dabei bleibt der am Durchflußregler anstehende Druckwert erhalten. Beim Erreichen des vorgewählten Solldruckes wird der momentane Durchfluß-Meßwert in der elektronischen Steuerschaltung gespeichert und zur Bestimmung des intraabdominalen Druckes nach einer bestimmten Formel verwendet, in welche mehrere Meßparameter eingehen. Diese Ermittlung des intraabdominalen Druckes über die Flowgröße und den Rückstaudruck am Vorrichtungsausgang ist besonder bei kleinem Druck und großen Flow am unsichersten. Gerade dies ist aber die häufigste Situation in der normalen Anwendung dieser Vorrichtung.

Der Erfindung liegt von daher die Aufgabe zugrunde, eine Vorrichtung der gattungsgemäßen Art ohne geräuscherzeugende Magnetventile und mit möglichst wenigen, in der Gasleitung angeordneten Bauelementen zu schaffen, mit welcher der intraabdominale Druck ohne aufwendige Umschaltung genauestens gemessen und der Flow genauestens gesteuert werden kann.

Die Lösung dieser Aufgabe ergibt sich aus den kennzeichnenden Merkmalen des Anspruches 1. Gegenüber der bekannten Vorrichtung bestehen Wirtschaftlichkeits-und Sicherheits-Vorteile im genauen, unmittelbaren Messen des intraabdominalen Druckes ohne aufwendige Umschaltung und ohne geräuscherzeugende Magnetventile.Ein wesentlicher Vorteil der erfindungsgemäßen Vorrichtung ist die kontinuierliche, automatisierte Insufflation, wozu nicht, wie bei den bisher bekannten Vorrichtungen, die Gaszufuhr abgestellt, sondern der Systemdruck variiert wird. Dadurch werden der intraabdominale Solldruck schneller erreicht und plötzliche Gasverluste besser abgefangen.

In Abhängigkeit vom vorgewählten Solldruck und dem elektrischen Signal des Durchflußmessers wird der elektrisch steuerbare Druckminderer mittels der elektronischen Steuerschaltung gesteuert. Der Druckminderer erhält seinen Vordruck aus einem Druckbehälter oder aus einem weiteren vorgeschalteten Druckminderer mit höherem Ausgangsdruck als 50 mmHg, wobei der Steuerbereich des elektrisch steuerbaren Druckminderers zwischen 0 und 50 mmHg liegt. Nach dem Einschalten der Vorrichtung wird der steuerbare Druckminderer mittels der elektronischen Steuerschaltung auf den eingestellten Solldruck gefahren. Übersteigt das am Durchflußmesser ermittelte Flowsignal einen in der Steuerschaltung voreingestellten Wert , so steuert die elektronische Steuerschaltung den Ausgangsdruck des Druckminderers auf den Maximalwert von 50 mmHg. Nach dem Ende einer vom Taktgenerator vorgegebenen Taktperiode wird der Ausgangsdruck des Druckminderers, gesteuert von der elektronischen Steuerschaltung, wieder auf den Sollwert heruntergefahren. Das vom Durchflußmesser erhaltene Flowsignal wird daraufhin überprüft, ob der Flow beim Solldurck noch den vorgegebenen Wert übersteigt. Sofern der Flow beim vorgegebenen Wert den Solldruck übersteigt, fährt die elektronische steuerschaltung den elektrisch steuerbaren Druckminderer wieder für eine Taktperiode auf den Ausgangsdruck von 50 mmHg hoch. Sofern der Flow beim Solldruck den vorgegebenen Wert nicht übersteigt, steuert die elektronische Steuerschaltung den elektrisch steuerbaren Druckminderer weiter nur auf den Sollwert und überwacht das Signal des Durchflußmessersim Hinblick darauf, ob dieses den vorgegebenen Wert übersteigt. Sofern dieser Wert gelegentlich überstiegen wird, wird der steuerbare Druckminderer wieder für eine Taktperiode bis auf einen Ausgangsdruck von 50 mmHg hochgefahren. Der entstehende intraabdominale Druck wird mit dem Druckmesser dann gemessen, wenn der steuerbare Druckminderer in seinem Ausgangsdruckwert so weit heruntergefahren ist, daß der mit dem Durchflußmesser gemessene Flow gerade den Wert Null erreicht hat. Dann ist der intraabdominale Druck gleich dem gesteuerten Druck. Dieser wird optisch angezeigt. Auch der Flow wird sowohl optisch als auch akustisch angezeigt. Die Meßtaktfrequenz des Taktgenerators ist unabhängig von der Regelfrequenz der Vorrichtung. Zur simultanen Eigenkontrolle wird der vom Druckmesser gemessene Druck in der elektronischen Steuereinrichtung mit dem Druckwert verglichen, der beim Flow vom Wert Null angezeigt wird.

Weitere vorteilhafte Ausgestaltungen der Erfindung ergeben sich aus den Unteransprüchen.

Die Erfindung ist nachfolgend anhand eines in den Zeichnungen dargestellten Ausführungsbeispieles einer Vorrichtung zum Insufflieren von Gas näher erläutert.Es zeigen:

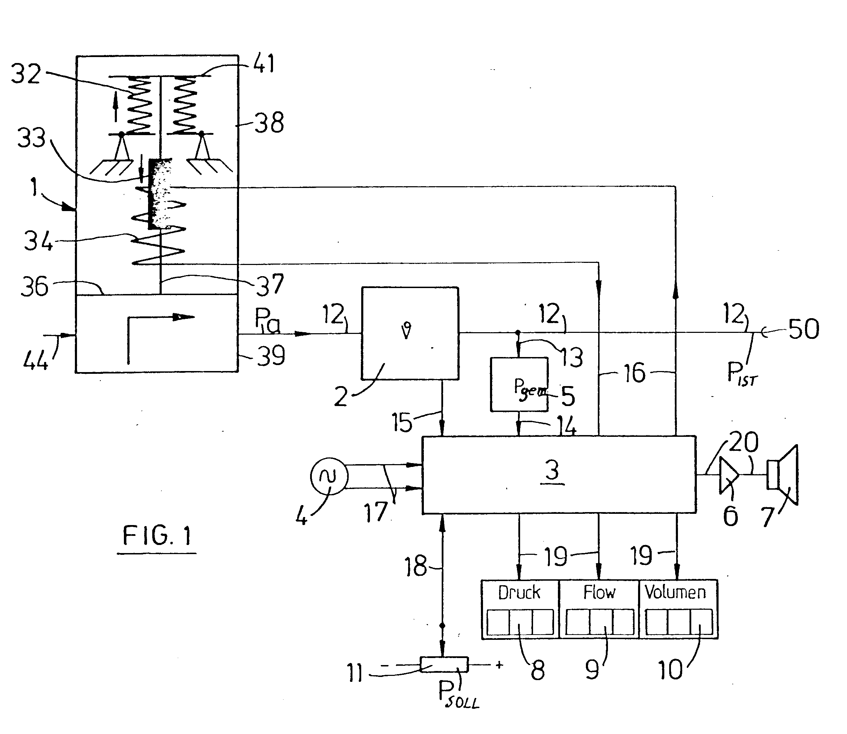

Fig. 1 ein Schaltschema der gesamten Vorrichtung,
Fig. 2 die elektronische Steuerschaltung der Vorrichtung,
Fig.3 einen Axialschnitt durch den elektrisch steuerbaren Druckminderer ,
Fig. 4 A bis C Kurvenverläufe des Ausgangsdruckes $P_a$ des Druckminderers über der Zeit in verschiedenen Betriebszuständen ,
Fig. 5 eine Regelzyklus - Flußdiagramm und
Fig. 6 ein Meß- und Selbstüberwachungs- Flußdiagramm.

Die Vorrichtung zum Insufflieren von Gas, insbesondere $CO_2$, in den menschlichen oder tierischen Körper, insbesondere zur Laparoskopie, mittels eines Insufflationsinstrumentes 50 insbesondere eine einläufige Veress-Nadel, umfaßt entlang einer Gasleitung 12 hintereinander einen steuerbaren Druckminderer 1, einen Durchflußmesser 2 und einen, in einer abgezweigten Gasleitung 13 geschalteten Druckmesser 5. Zwischen dem Durchflußmesser 2, welcher insbesondere auf Differenzdruckbasis arbeitet, und die zum Druckmesser 5 führende Abzweigleitung 13 kann ein nicht näher dargestelltes Überdruckventil eingeschaltet sein. Weitere Bauelemente sind in der Gasleitung 12 nicht vorgesehen, so daß diese geringste Strömungswiderstände aufweist.

Eine elektronische Auswerte- und Steuerschaltung 3 ist über eine Steuerleitung 14 mit dem Druckmesser 5 und über eine Steuerleitung 15 mit dem Durchflußmesser 2 sowie ferner über Steuerleitungen 16 mit dem steuerbaren Druckminderer 1 elektrisch verbunden. Die Steuerschaltung 3 ist ferner über Steuerleitungen 17 mit einem taktgebenden Taktgenerator 4 und über eine Steuerleitung 18 mit einem Solldruckgeber 11 verbunden. Ferner führen Steuerleitungen 19 zu LED-Anzeigeelementen 8,9 und 10 für den Druck bzw. den Flow bzw. das Volumen. Schließlich führt eine Steuerleitung 20 von der elektronischen Steuerschaltung 3 über einen Audio-Verstärker 6 zu einem Lautsprecher 7 zur Ausgabe von akustischen Flow-Signalen.

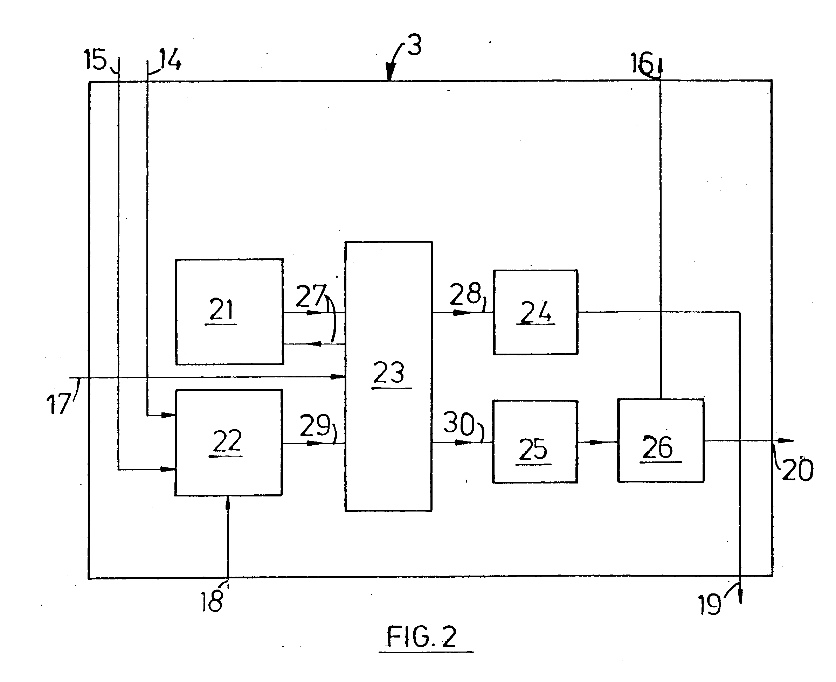

Die elektronische Auswerte- und Steuerschaltung 3 ist in Fig. 2 in einem Blockschaltbild näher dargestellt. Die Steuerschaltung 3 weist eine zentrale Prozessorschaltung ( CPU) 23 auf, an welche eingangsseitig über Steuerleitungen 27 ein Programmspeicher 21, über die Steuerleitung 17 der taktgebende Taktgenerator 4 und ein mit dem Durchflußmesser 2 über die Steuerleitung 15 und dem Druckmesser 5 über die Steuerleitung 14 sowie mit dem Solldruckgeber 11 über die Steuerleitung 18 verbundener 3-kanaliger A/D -Konverter 22 über die Steuerleitung 29 angeschlossen sind. Ausgangsseitig sind an die elektronische Auswerte- und Steuerschaltung 23 über eine Steuerleitung 28 ein LED-Treiber 24 zur Anzeige von Druck, Flow und Volumen an den LED- Anzeigen 8, 9 bzw. 10 über die Steuerleitung 19 und über die Steuerleitung 30 ein 2-kanaliger D/A-Konverter 25 mit angeschlossener Endstufe 26 angeschlossen, welche zur Steuerung des elektrisch steuerbaren Druckminderers 1 über die Steuerleitungen 16 und zur Ausgabe von Signalen über den mittels der Steuerleitung 20 angeschlossenen Lautsprecher 7 dient.

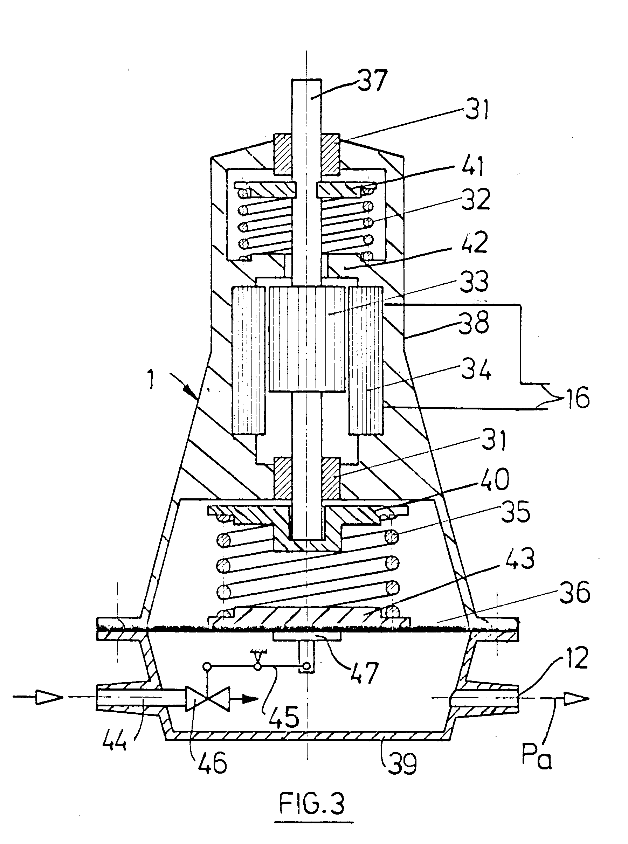

Der elektrisch steuerbare Druckminderer 1 ist in seiner konstruktiven Bauart in Fig. 3 dargestellt. Innerhalb eines zweiteiligen Gehäuses 38, 39 ist eine Steuermembrane 36 eingespannt, deren Unterseite ein in die Gasleitung 12 eingesetztes Steuerventil 46 über einen mittig am Gehäuseteil 39 gelagerten zweiarmigen Hebel 45 steuert, welcher mit einem Ende an einer Membranenplatte 47 und mit seinem anderen Ende am nicht näher dargestellten Steuerteil des Steuerventiles 46 angreift. Der untere Gehäuseteil 39 wird eingangsseitig über eine Gasleitung 44 von einem nicht dargestellten Druckbehälter über einen eventuelle vorgeschalten weiteren Druckminderer mit höherem Ausgangsdruck als 50 mmHg gespeist. Der Steuerbereich des elektrisch steuerbaren Druckminderers 1 liegt bei einem in die Gasleitung 12 eingespeisten Ausgangsdruck $P_a$ zwischen O und 50 mmHg. Im oberen Gehäuseteil 38 ist eine der Steuermembrane 36 zugewandte Aufnahmeöffnung für eine Druckmindererfeder 35 vorgesehen, welche mit einem Federgegenlager 43 an der Steuermembrane 36 anliegt. Das von der Steuermembrane 36 abgewandte Federgegenlager 40 ist von der Achse 37 eines Magnetkernes 33 beaufschlagt, welche Achse 37 in oberen und unteren axialen Gleitlagern 31 geführt ist. Der Magnetkern 33 ist von einer im oberen Gehäuseteil 38 gelagerten Magnetspule 34 umgeben, welche über die Steuerleitungen 16 mit der elektronischen Auswerte- und Steuerschaltung 3 elektrisch verbunden ist. Die Achse 37 ist ferner über eine sich zwischen einem Gehäusebund 42 und einem an der Achse 37 festgelagerten Federgegenlager 41 erstreckende Rückstellfeder 32 beaufschlagt. Der elektrisch steuerbare Druckminderer 1 arbeitet nach dem Prinzip Druckgaskraft gleich Federgegenkraft. Der resultierende Weg der Steuermembrane 36 öffnet und schließt das Steuerventil 46. Der aus Magnetkern 33 und Magnetspule 34 gebildete Elektromagnet positioniert die Druckmindererfeder 35 über deren Federgegelager 40. Daraus resultierend ändert sich die auf die Steuermembrane 36 wirkende Kraft der Druckmindererfeder 35.

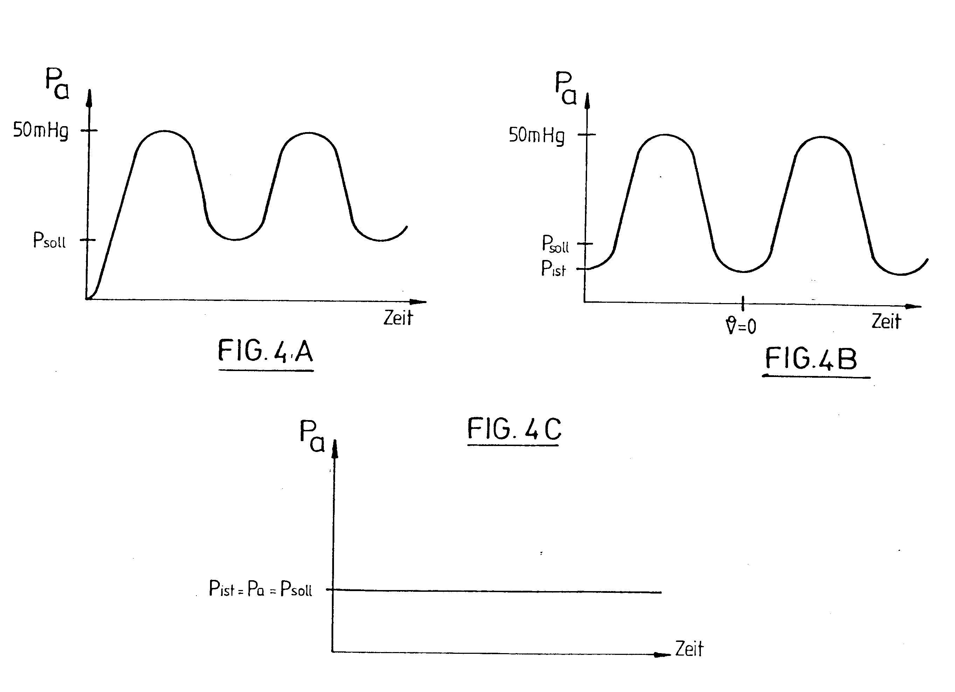

Gemäß Fig. 4A wird nach dem Einschalten der Vorrichtung der Ausgangsdruck $P_a$ des steuerbaren Druckminderers 1 auf den mit dem Solldruckgeber 11 eingestellten Solldruck $P_{SOLL}$ gefahren. Sofern das vom Durchflußmesser 2 ermittelte Flowsignal einen in der Steuerschaltung voreingestellten Wert übersteigt, so steuert die Steuerelektronik 3 den Ausgangsdruck des steuerbaren Druckminderers 1 auf den maximalen Ausgangsdruck $P_a = 50$ mmHg. Nach dem Ende einer vom taktgebenden Taktgenerator 4 vorgegebenen Taktperiode ( Zeit) steuert die Steuerschaltung 3 den Druck $P_a$ wieder auf den Sollwert $P_{SOLL}$ herunter. Das Flowsignal wird wiederum vom Durchflußmesser 2 überprüft. Es wird geprüft, ob der Flow beim Erreichen des Solldrucks $P_{SOLL}$ den vorgegebenen Wert noch übersteigt. Sofern der vorgegebene Wert noch überstiegen wird, fährt die Steuerelektronik 3 den steuerbaren Druckminderer 1 wieder auf den maximalen Ausgangsdruck von 50 mmHg für eine vom Taktgenerator vorgegebene Taktperiode. Sofern der Flow beim Solldruck $P_{SOLL}$ den vorgegebenen Wert nicht übersteigt ( Fig. 4B), steuert die Steuerelektronik 3 den steuerbaren Druckminderer 1 weiter nur auf den Sollwert und überwacht das Signal des Durchflußmessers 2, ob dieses den vorgegebenen Wert übersteigt. Sofern dieser Wert gelegentlich überstiegen wird, wird der steuerbare Druckminderer 1 für eine weitere Taktperiode des Taktgenerators 4 auf $P_a = 50$ mmHg hochgefahren. Der entstehende intraabdominale Druck wird mit dem Druckmesser 5 dann gemessen, wenn der steuerbare Druckminderer 1 soweit heruntergefahren ist, daß der mit dem Durchflußmesser 2 gemessene Flow gerade den Wert Null erreicht hat, wie es in Fig. 4B mit dem Wert $\triangledown$ gleich Null dargestellt ist. Dann ist der intraabdominale Druck $P_{IST}$ gleich dem am Anzeigeelement 8 optisch angezeigten Druck. Der Flow wird sowohl am Anzeigeelement 9 angezeigt als auch akustisch mittels des Lautsprechers 7 ausgegeben. Die Meßtaktfrequenz der Vorrichtung ist unabhägig von der Regelfrequenz. Zur simultanen Eigenkontrolle der Vorrichtung wird der mittels des Druckmessers 5 gemessene Druck in der Steuerelektronik 3 mit dem Druckwert verglichen, der bei dem mit dem Durchflußmesser 2 beim Flow vom Wert Null gemessenen Druckwert entspricht.

Die Vorrichtung hat somit bei kontinuierlichem Flow drei verschiedene Arbeitszustände:

### 1. Einen reinen Füllvorgang

Dieser läuft, wie vorher bereits beschrieben, gemäß Fig. 4A ab, wobei ständig vom Durchflußmesser 2 die Werte für den Druck, den Flow und das Volumen von der Steuerschaltung 3 abgenommen und angezeigt werden.

### 2. Aktuelle Druckmessung

Diese erfolgt durch ein Herunterfahren des steuerbaren Druckminderers 1 auf den Flow vom Wert Null gemäß Fig. 4B. Dann entspricht der am Druckmesser 5 angezeigte Druck dem tatsächlichen intraabdominalen Druck, d.h. dem Druck $P_{IST}$. Dies erfolgt nur zum Messen, nicht jedoch zum Regeln der Insufflation.

### 3. Prüfzyklus

Sobald der Ausgangsdruck $P_a$ des Druckminderers 1 auf den Druckwert $P_{IST}$ heruntergefahren ist, kann die Steuerelektronik 3 den vom Druckmesser 5 ermittelten Druck und den gemessenen Druck mit dem apparativen Druck vergleichen, es erfolgt somit eine Eigenkontrolle des gemessenen Druckes ( Fig. 4C).

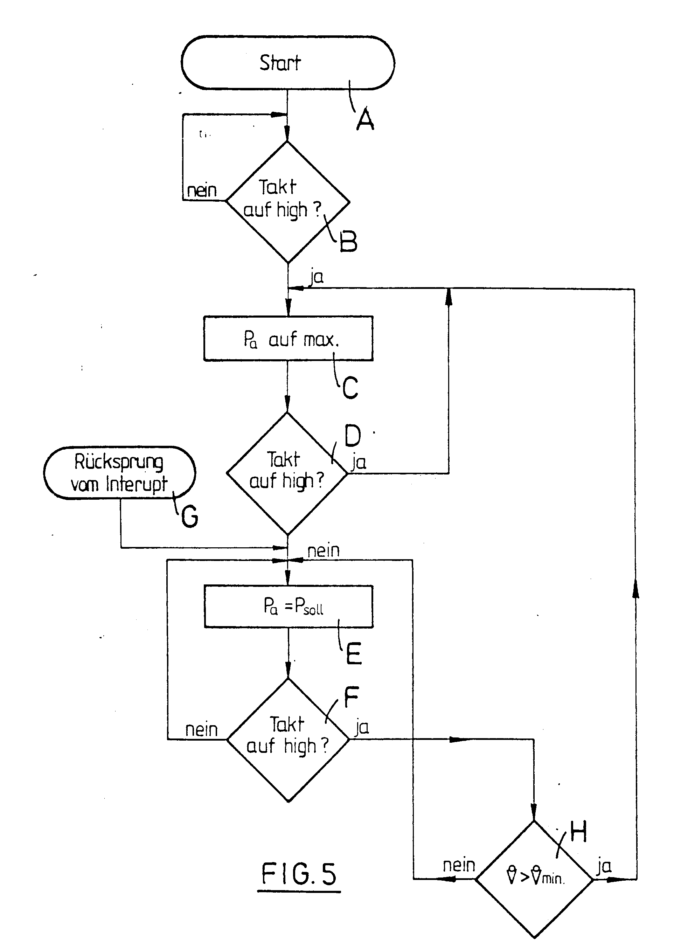

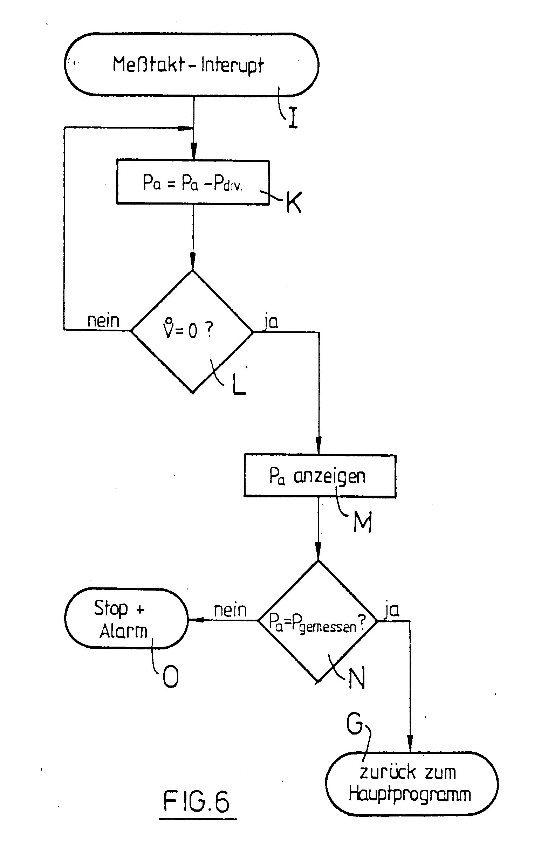

In den Figuren 5 und 6 werden Regelzyklus- bzw. Meß - und Selbstüberwachungs-Flußdiagramme dargestellt. Hierzu werden nachfolgend Erläuterungen gegeben. Block A " Start" bedeutet Einschalten der Vorrichtung. Im Block B erfolgt die Überprüfung des Taktes des taktgebenden Taktgenerators 4. Im Block C erfolgt die Einstellung des Ausgangsdruckes $P_a$ des elektrisch steuerbaren Druckminderers 1 auf maximalen Ausgangsdruck

$P_a$ gleich 50 mmHg. Im Block D erfolgt wiederum eine Prüfung des Taktes des Taktgenerators 4. Im Block E erfolgt die Einstellung des Ausgangsdruckes $P_a$ des einstellbaren Druckminderers 1 auf den am Sollwertgeber 11 eingestellten Solldruck $P_{SOLL}$. Im Block F erfolgt wieder eine Überprüfung des Taktes des Taktgenerators 4. Im Block H erfolgt eine Überprüfung des Flow $\dot{V}$. Ist dieser größer als der kleinste Flow $\dot{V}_{MIN}$, wenn der Istdruck $P_{IST}$ ein wenig kleiner ist als der Solldruck $P_{SOLL}$, so erfolgt eine Rückstellung zu Block C. Ist $\dot{V}$ kleiner $\dot{V}_{MIN}$, so erfolgt eine Rückstellung zu Block E. Der Druck $P_{IST}$ bedeutet den aktuellen Druck im Abdomen, d.h. den intraabdominalen Druck. Der Block G bezieht sich auf das Meß- und Selbstüberwachungs-Flußdiagrammgemäß Fig. 6.

Die Auslösung der Selbstüberwachung erfolgt im Block I. Im Block K erfolgt eine Reduzierung des Ausgangsdruckes $P_a$ des steuerbaren Druckminderers 1 um eine Druckdifferenz $P_{DIV}$ von ca. 2 mmHg. Im Block L erfolgt eine Überprüfung, ob der Flow $\dot{V}$ den Wert Null erreicht hat. Wenn ja, wird im Block M der Ausgangsdruck $P_a$ des regelbaren Druckminderers 1 angezeigt. Im Block N wird überprüft, ob der angezeigte Ausgangsdruck $P_a$ dem mit dem Druckmesser 5 gemessenen Druck $P_{gemessen}$ entspricht. Wenn dies nicht zutrifft, erfolgt der Schritt zum Block O, gemäß welchem die Vorrichtung gestoppt und ein Alarmausgelöst wird. Wenn der Ausgangsdruck $P_a$ am regelbaren Druckminderer 1 dem vom Druckmesser 5 gemessenen Druck $P_{gemessen}$ entspricht, so erfolgt der Schritt zum Block G, d.h. zurück zum Hauptprogramm gemäß dem Regelzyklus-Flußdiagramm nach Fig. 5.

Die Voreinstellung des Wertes für den gewünschten Flow erfolgt in dem Programmspeicher 21 der Steuerschaltung 3.

**Patentansprüche**

1. Vorrichtung zum Insufflieren von Gas, insbesondere $CO_2$, in den menschlichen oder tierischen Körper, insbesondere zur Laparoskopie, mittels eines Insufflationsinstrumentes, z.B. einer einläufigen Veress-Nadel, mit entlang einer Gasleitung (12) hintereinander angeordnetem Druckminderer (1), Durchflußmesser (2) und Druckmesser (5) und mit einer mit dem Durchfluß- und dem Druckmesser über eine Steuerleitung (16) verbundenen Steuerschaltung (3),
   **dadurch gekennzeichnet**,
   daß der Druckminderer (1) zur Variation des Arbeitsdruckes ($P_a$) in der Gasleitung (12) von der Steuerschaltung (3) über eine Steuerleitung (16) in Abhängigkeit von einem Taktgeber (4) und einem Solldruckgeber (11) steuerbar ist.

2. Vorrichtung nach Abspruch 1, dadurch gekennzeichnet, daß die Auswerte- und Steuerschaltung (3) eine zentrale Pozessorschaltung (CPU) (23) aufweist, an welche eingangsseitig ein Programmspeicher (21), der Taktgeber (4) und ein mit dem Durchflußmesser (2), dem Druckmesser (5) und dem Solldruckgeber (11) verbundener 3-kanaliger A/D-Konverter (22) und ausgangsseitig ein LED-Treiber (24) zur Anzeige von Druck, Flow und Volumen an LED-Anzeigen (8 bzw. 9 bzw. 10) und ein 2-kanaliger D/A-Konverter (25) mit angeschlossener Endstufe (26) zur Steuerung des elektrisch steuerbaren Druckminderers (1) und zur Ausgabe von Signalen über einen angeschlossenen Lautsprecher (7) angeschlossen sind.

3. Vorrichtung nach Anspruch 1 oder 2, dadurch gekennzeichnet, daß der steuerbare Druckminderer (1) eine Steuermembran (36) aufweist, deren Unterseite ein in die Gasleitung (12) eingesetztes Steuerventil (46) betätigt, und deren Oberseite mit einer Druckmindererfeder (35) verbunden ist, deren der Membrane (36) abgewandtes Federgegenlager (40) von der Achse (37) eines, in eine von der Steuerschaltung (3) steuerbare Magnetspule (34) eintauchenden, mit einer Rückstellfeder (32) versehenen Magnetkerns (33) beaufschlagt ist.

**Claims**

1. A device for insufflating gas, in particular $CO_2$, into a human or animal body, in particular for laparoscopy, by means of an insufflation instrument, in particular a single-channeled Veress needle, comprising along a gas line (12) a controllable pressure reducer (1), a flowmeter (2) and a pressure meter (5), and comprising a control circuitry (3) connected with the said flowmeter and the said pressure meter over a control line (16), characterized by that the pressure reducer (1) is controllable, for varying the operating pressure ($P_a$) in the gas line (12), by the control circuitry (3) over a control line (16), depending from a clock generator (4) and from a desired pressure generator (11).

2. A device according to claim 1, characterized by that the evaluator and control circuitry (3) comprises a central processor unit (CPU) (23), to the inputs of which a programme memory (21), the clock generator (4) and a 3-channel A/D converter (22) connected with the flowmeter (2), with the pressure meter (5) and with the desired pressure generator (11) are connected, and on the output side of which a LED driver (24) for indicating pressure, flow or volume at LED displays (8, 9 or 10, resp.), and a 2-channel D/A converter (25) with connected end stage (26) are provided, for controlling the electrically controllable pressure reducer (1) and for outputting signals over a connected loudspeaker (7).

3. A device according to claims 1 or 2, characterized by that the controllable pressure reducer (1) comprises a control diaphragm (36), the lower side of which actuates a control valve (46) inserted into the gas line (12), and the upper side of which is connected with a pressure reducer spring (35), the spring counter-support (40) thereof turned away from the diaphragm (36) being loaded by the shaft (37) of a magnetic core (33), surrounded by a magnetic coil (34) being controlled by the control circuitry (3), and the magnetic core being further provided with a reset spring (32).

**Revendications**

1. Dispositif d'insufflation de gaz, en particulier $CO_2$, dans le corps humain ou d'un animal, en particulier pour la laparoscopie, au moyen d'un instrument d'insufflation, en particulier une aiguille Veress à un canal, comportant le long d'une conduite de gaz (12) l'un derrière l'autre un détendeur commandable (1), un débitmètre (2) et un indicateur de pression (5) et un circuit de commande (3) raccordé audit débitmètre et indicateur de pression par l'intermédiaire d'une ligne de commande (16), caractérisé en ce que le détendeur (1) est commandable, pour la variation de la pression de travail dans la conduite de gaz (12), par le circuit de commande (3) par l'intermédiaire d'une ligne de commande (14) en dépendance d'un rythmeur (4) et d'un dispositif fournissant la pression désirée.

2. Dispositif selon la revendication 1, caractérisé en ce que le circuit de commande (3) comporte un processeur (CPU) (23), aux entrées duquel une mémoire de programme (21), le rythmeur (4) et un convertisseur anologique-numérique à 3 canaux (22) raccordé au débitmètre (2), à l'indicateur de pression (5) et au composant (11) fournissant la pression désirée sont raccordés, et à la sortie duquel un circuit d'attaque LED (24) pour l'indication de pression, débit et volume aux affichages LED (8, 9 ou 10) et un convertisseur numérique-analogique (25) à 2 canaux avec étage de sortie (26), qui sert de commande du détendeur (1) commandable et de sortie de signaux par l'intermédiaire d'un haut-parleur (7) raccordé sont raccordés.

3. Dispositif selon la revendication 1 ou 2, caractérisé en ce que le détendeur commandable (1) comporte un diaphragme de commande (36), la face inférieure duquel commandant une vanne-pilote (46) intercalée dans la conduite de gaz (12), et la face supérieure duquel étant raccordée avec un ressort (35) du détendeur, sur le contre-support (40) duquel agissant l'axe (37) d'un noyau magnétique (33), entouré d'une bobine d'électro-aimant (34) commandable par le circuit de commande (3) et pourvu d'un ressort de rappel (32).

FIG. 1

EP 0 243 295 B1

FIG.2

FIG.3

FIG. 4.A

FIG.4B

FIG. 4C

FIG.5

Meßtakt - Interupt

I

$P_a = P_a - P_{div.}$

K

$\mathring{V} = 0$ ?  nein  ja

L

$P_a$ anzeigen

M

Stop + Alarm  nein  $P_a = P_{gemessen}$ ?  ja

O  N

G

zurück zum Hauptprogramm

## FIG.6